# EUROPEAN PATENT APPLICATION

(11) **EP 3 196 643 A1**
(43) Date of publication of application: **26.07.2017**
(21) Application number: 16305060.2
(22) Date of filing: 22.01.2016
(51) Int. Cl.: G01N 33/00, G01N 1/02, G01N 1/22

(54) **A HEAD MOUNTED DEVICE COMPRISING AN ENVIRONMENT SENSING MODULE**

(71) Applicant: Essilor International, 94220 Charenton Le Pont (FR)
(72) Inventor: BOULAROT, Adrien, 94227 Charenton-Le-Pont Cedex (FR); LORE, Marie, 94227 Charenton-Le-Pont Cedex (FR)
(74) Representative: Cabinet Novitech

(57) **Abstract**

A head mounted device comprising an environment sensing module configured to sense at least one parameter of the environment of the user of the head mounted device, the sensing module comprising at least:
- a sensor configured to sense a parameter of the environment of the user of the head mounted device,
- a communication component configured to send data sense by the sensor indicative of the parameter to an analyzing module,
wherein the parameter relates to the air in the direct environment of the user and/or the electromagnetic wave in the direct environment of the user and/or the acoustic wave in the direct environment of the user.
Environmental analyzing module for analyzing an environment parameter of a user of the head mounted device. Environmental parameter mapping system comprising plural such head mounted devices.

## Description

### FIELD OF THE INVENTION

The invention relates to a head mounted device comprising an environment sensing module configured to sense at least one parameter of the environment of the user of the head mounted device, an environmental analyzing module configured to analyze at least one parameter of the environment of a user, and an environmental parameter mapping system.

### BACKGROUND OF THE INVENTION

Some cities have installed air composition control devices around the city to sense air composition so as to provide a monitoring of air composition.

Such devices allow cities to alert when the concentration of a specific molecule in the air around the city is above a given value.

Although such devices provide good average result they have some drawbacks. The first of them is that the sampling may be larger, thus providing only an average result over the city. Therefore, the resolution of such system may be low and is usually limited to providing information over a wide area, such as a city or a neighborhood.

One object of the present invention is to provide such an improved environment sensing device

### SUMMARY OF THE INVENTION

To this end, the invention proposes a head mounted device comprising an environment sensing module configured to sense at least one parameter of the environment of the user of the head mounted device, the sensing module comprising at least:
- a sensor configured to sense a parameter of the environment of the user of the head mounted device,
- a communication component configured to send data sense by the sensor indicative of the parameter to an analyzing module,
wherein the parameter relates to the air in the direct environment of the user and/or the electromagnetic wave in the direct environment of the user and/or the acoustic wave in the direct environment of the user.

Advantageously, having the sensing module mounted on a head mounted device allows sensing a parameter in the direct environment of the user.

Prior art sensing devices are usually placed outside or at least in public places. Therefore, although most people spend their time indoor, all private places, such as stores and homes are usually not monitored by such devices. It has been observed that the air composition may be very different inside a home from outside.

The head mounted device according to the invention allows providing an accurate sensing of at least one parameter in the air in the direct environment of the user regardless to the fact of being inside or outside.

Furthermore, having a head mounted sensor allows sensing parameter very close to the eyes, nose, mouth, ears and brain of the user, thus providing more relevant data concerning air, the electromagnetic wave and acoustic wave than if carried in the bag or pocket or even wrist of the user.

According to further embodiments which can be considered alone or in combination:
- the sensor is adapted to sense the properties and/or composition of the air around the user, for example the presence and/or the quantity of allergen and/or of a specific chemical compound and/or of microparticles in the air around the user; and/or
- the head mounted device according to the invention further comprises a spectacle frame and wherein the sensor is mounted on the bridge and/or pad and/or brace bar of the spectacle frame; and/or
- the sensor is adapted to sense the intensity and/or frequency of the sound in the environment of the user; and/or
- the head mounted device further comprises a spectacle frame and wherein the sensor is mounted on the side of the spectacle frame; and/or
- the sensor is adapted to sense the intensity and/or wavelength and/or direction of electromagnetic radiations in the environment of the user; and/or
- the head mounted device further comprises a geolocation module configured to determine geolocation data relative to the geolocation of the user of the head mounted device, and wherein the communication component is configured to send geolocation data together with the data indicative of the parameter of the environment to a mapping module; and/or
- the head mounted device further comprises a mapping module comprising:
   o a communication module configured to receive from the communication component data indicative of the parameter sense by the sensor together with geolocation data,
   o a memory storing at least computer executable instructions; and
   o a processor for executing the computer executable instructions stored in the memory, wherein the computer executable instructions comprises instructions for processing the data indicative of the parameter of the environment of the user and the geolocation data to generate a mapping of the parameter of the environment; and/or
- the head mounted device further comprising an environment analyzing module comprising:
   o a communication element configured to receive from the communication component data indicative of the parameter sense by the sensor,
   o a memory storing at least computer executable instructions; and
   o a processor for executing the computer executable instructions stored in the memory, wherein the computer executable instructions comprises instructions for processing the data indicative of the parameter of the environment of the user to generate environment information indicative of to the air composition and/or the electromagnetic radiations and/or the sound of the environment of the user
- the environment information comprises an alert information and/or a recommendation, and/or an activation parameter; and/or
- the communication element is further configured to receive from a distant entity, for example a mapping module, a mapping of a mapping parameter of the environment of the user, the mapping parameter relating to the air composition and/or the electromagnetic radiations and/or the sound of the environment of the user, and the computer executable instructions further comprises instructions for processing the mapping upon generating environment information; and/or
- the parameter sensed by the sensor and the mapping parameter relate to the air composition in the environment of the user; and/or
- the parameter sensed by the sensor and the mapping parameter relate to the electromagnetic radiations in the environment of the user; and/or
- the parameter sensed by the sensor and the mapping parameter relate to the sound in the environment of the user; and/or
- the head mounted device further comprises an output unit configured to communicate with the environment analyzing module and to output a signal to the user based on the environment information received from the environment analyzing module.

The invention further relates to an environmental analyzing module configured to analyze at least one parameter of the environment of a user of a head mounted device according to the invention, the module comprising:
- a communication element configured to receive from the communication component data indicative of the parameter sense by the sensor of the head mounted device,
- a memory storing at least computer executable instructions; and
- a processor for executing the computer executable instructions stored in the memory, wherein the computer executable instructions comprises instructions for processing the data indicative of the parameter of the environment of the user to generate environment information indicative of to the air composition and/or the electromagnetic radiations and/or the sound of the environment of the user.

The environment information may comprise alert information and/or a recommendation, and/or an activation parameter.

The invention also relates to an environmental parameter mapping system comprising a plurality of head mounted devices according to the invention, wherein each head mounted device further comprises a geolocation module configured to determine geolocation data relative to the geolocation of the user of the head mounted device, and wherein the communication component is configured to send geolocation data together with the data indicative of the parameter of the environment to a mapping module and each head mounted device is arranged to send geolocation data together with data indicative of the parameter of the environment to the mapping module, for example a common mapping module, configured to determine a mapping of the parameter over the zone in which the plurality of head mounted device is located.

The head mounted devices may be arranged to communicate geolocation data and/or data indicative of the parameter of the environment to each other.

The invention further relates to the use of a mapping determined by an environmental parameter mapping system according to the invention for providing a service. For example, the mapping may be used to determine real estate pricing. For example, the mapping may indicate that such street or corner or block is less or more polluted or noisy or comprises a greater or less density of electromagnetic waves than average. Such indication may be taken into account when determining the price of real estate.

The invention also relates to a method for monitoring at least one parameter of the environment of a user of a head mounted sensing device according to the invention, the method comprising:
- an environment data receiving step S1, during which environment data indicative of at least one parameter of the environment of the user are received form the head mounted sensing device, the at least one parameter relates to the air in the direct environment of the user and/or the electromagnetic wave in the direct environment of the user and/or the acoustic wave in the direct environment of the user,
- an environment information generating step S2, during which an environment information is generated based at least on the received environment data.

The method of the invention may further comprise prior to the environment data receiving step S1 a sensing step during which at least one parameter relating to the air in the direct environment of the user and/or the electromagnetic wave in the direct environment of the user and/or the acoustic wave in the direct environment of the user is sense by the sensor of the head mounted sensing device according to the invention.

The method of the invention may further comprise
- a geolocation data receiving step during which geolocation data relative to the geolocation of the user are received, and
- a mapping generating step during which a mapping of the at least one parameter of the environment is generated based on the received environment data and geolocation data.

The invention further relates to a computer program product comprising one or more stored sequences of instructions that are accessible to a processor and which, when executed by the processor, causes the processor to carry out at least the steps of the method according to the invention.

The invention also relates to a computer-readable storage medium having a program recorded thereon; where the program makes the computer execute at least the steps of the method of the invention.

The invention further relates to a device comprising a processor adapted to store one or more sequence of instructions and to carry out at least steps of the method according to the invention.

Unless specifically stated otherwise, as apparent from the following discussions, it is appreciated that throughout the specification discussions utilizing terms such as "computing", "calculating", or the like, refer to the action and/or processes of a computer or computing system, or similar electronic computing device, that manipulate and/or transform data represented as physical, such as electronic, quantities within the computing system's registers and/or memories into other data similarly represented as physical quantities within the computing system's memories, registers or other such information storage, transmission or display devices.

Embodiments of the present invention may include apparatuses for performing the operations herein. This apparatus may be specially constructed for the desired purposes, or it may comprise a general purpose computer or Digital Signal Processor ("DSP") selectively activated or reconfigured by a computer program stored in the computer. Such a computer program may be stored in a computer readable storage medium, such as, but is not limited to, any type of disk including floppy disks, optical disks, CD-ROMs, magnetic-optical disks, read-only memories (ROMs), random access memories (RAMs) electrically programmable read-only memories (EPROMs), electrically erasable and programmable read only memories (EEPROMs), magnetic or optical cards, or any other type of media suitable for storing electronic instructions, and capable of being coupled to a computer system bus.

The processes and displays presented herein are not inherently related to any particular computer or other apparatus. Various general purpose systems may be used with programs in accordance with the teachings herein, or it may prove convenient to construct a more specialized apparatus to perform the desired method.

The desired structure for a variety of these systems will appear from the description below. In addition, embodiments of the present invention are not described with reference to any particular programming language. It will be appreciated that a variety of programming languages may be used to implement the teachings of the inventions as described herein.

### BRIEF DESCRIPTION OF THE DRAWINGS

Embodiments of the invention will now be described, by way of example only, and with reference to the following drawings in which:
- Figure 1 is a schematic representation of a head mounted device according to the invention,
- Figure 2 is a schematic representation of head mounted device communicating with an environment analyzing module, and
- Figure 3 represents an environmental parameter mapping system according to the invention.

### DETAILED DESCRIPTION OF PREFERRED EMBODIMENTS

Elements in the figures are illustrated for simplicity and clarity and have not necessarily been drawn to scale. For example, the dimensions of some of the elements in the figure may be exaggerated relative to other elements to help improve the understanding of the embodiments of the present invention.

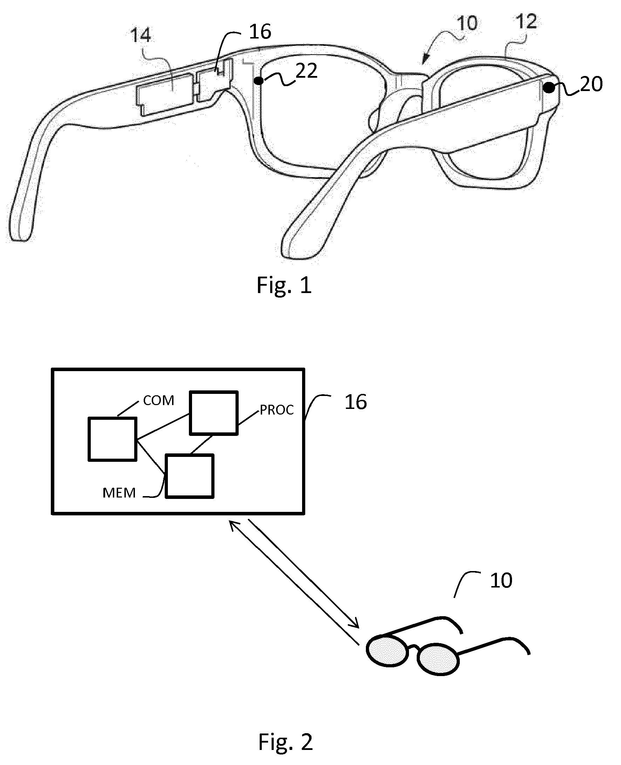

As illustrated on figure 1, the invention relates to a head 10 comprising an environment sensing module configured to sense at least one parameter of the environment of the user of the head mounted device.

The head mounted device may typically comprises a spectacle frame 12 with a sensing module comprising sensors 20 and 22 and a communication component 14.

The sensing module comprising at least a sensor, for example a plurality of sensors, configured to sense a parameter of the environment of the user of the head mounted device.

The parameter typically relates to the air in the direct environment of the user and/or the electromagnetic wave in the direct environment of the user and/or the acoustic wave in the direct environment of the user.

In the sense of the invention the direct environment of the wearer is to be understood as the environment within a few meters from the user. Typically, the direct environment comprises the air the user can breathe and/or the sounds the user may hear and/or the electromagnetic waves the user may receive.

The head mounted device may comprise a sensor adapted to sense the properties and/or composition of the air in the direct environment of the user, for example within 5 meters of the user. The sensor may be adapted to sense the presence and/or quantity of allergen and/or of a specific chemical compound and/or microparticules in the air around the user. The sensor may be adapted to detect and/or quantify the presence of dust mites, specific odor, toxic gas such as CO or others.

The sensors may be a chemical or optical sensor such as a camera. The device according to the invention may comprise an RFID reader that could be used to determine the risk of allergies for the user when eating a specific food or taking a specific drug.

So as to increase the accuracy of such sensors they may be mounted on the bridge and/or pad and/or brace bar of the spectacle frame.

The sensors may be adapted to sense sounds in the direct environment of the user. For example, the sensor may be adapted to detect the intensity and/or frequency of the sounds in the environment of the wearer. Such sensor may typically be a microphone embedded in the spectacle frame. Such sensor may typically be placed on the side of the head mounted device so as to acquire sound information of the environment of the user.

The sensors may be adapted to sense the intensity and/or wavelength and/or direction of electromagnetic radiations in the environment of the user.

The sensors may be more specifically adapted to detect light electromagnetic radiations, typically for wave lengths comprised between 10 nm and 100 µm. Such sensors are typically luminance, photodiode light meter, camera.

Sensing the intensity and/or frequency of the light received by the user may be used to determine glare risk by over-exposure or under-exposure light.

The sensors may be specifically adapted to detect micro and/or radio waves, in particular the intensity and frequency of the received electromagnetic radiations. Suh sensors may be for example photodiodes and/or spectroportables, and/or electromagnetic radiation detectors.

The sensing module may further comprise temperature and/or humidity sensors to determine the temperature and/or humidity around the wearer.

The communication component 14 is configured to send data sense by the sensor indicative of the parameter to an analyzing module 16.

An environment analyzing module 16 according to the invention typically comprises:
- a communication element configured to receive from the communication component data indicative of the parameter sense by the sensor,
- a memory storing at least computer executable instructions and configured to store the received data; and
- a processor for executing the computer executable instructions stored in the memory.

The computer executable instructions typically comprises instructions for processing the data indicative of the parameter of the environment of the user to generate environment information indicative of to the air composition and/or the electromagnetic radiations and/or the sound of the environment of the user.

As represented on figure 1, the environment analyzing module 16 may be embedded in the head mounted device.

The environment analyzing module 16 may be part of a distant entity as illustrated in figure 2.

According to an embodiment of the invention the processing of the environment information is generated using statistical analysis of the collected data.

Statistics involves the collection, organization, analysis, interpretation, and/or presentation of measured/collected data. With advances in technology, more extensive and complex computing allows massive amounts of data to be collected, stored and/or processed. Further, methods for evaluating the data are numerous.

Statistical analysis can be employed to process and/or evaluate data sensed such as data indicative of the parameter of the environment of the user to generate environment information indicative of to the air composition and/or the electromagnetic radiations and/or the sound of the environment of the user. The two main types of statistics are descriptive and inferential statistics.

Descriptive statistics includes methods for organizing and summarizing collected data. These methods include, but are not limited to, graphs, tables, charts and measurements such as averages, percentiles, and measures of variation of the data. Data mining for pattern detection, machine learning and artificial intelligence methods, regression modeling and summary statistics can be employed in descriptive statistics.

Inferential statistics is based on methods for making conclusions about data collected based on the evaluation of a sample of the data. For example, predictions can be made regarding the entire set of data. An example of prediction can relate to the risk of allergies of the user based on data collected. Recommendations can be made to achieve or avoid such allergies.

Statistical methods such as regression analysis can be employed to analyze data. Regression analysis includes techniques for analyzing different variables to determine the relationship between one or more dependent variables and independent variables. For example, the analysis can be employed to determine how the value of a dependent variable changes when a value of one independent variable changes while keeping the values of other independent variables constant. Regression analysis can be employed for prediction and overlaps with the field of machine learning; a branch of artificial intelligence that employs algorithms to identify patterns in data and/or make predictions based on evaluated data.

Different models can be employed in regression analysis to model the relationship between two variables. Linear regression is a type of regression analysis. Linear regression models the relationship between a dependent variable and an independent variable using linear predictor functions. Unknown model parameters are estimated from the data on which linear regression is performed. Interpolation methods can be employed to perform prediction based on values within the set of collected data used for model-fitting while extrapolation can be employed to perform prediction based on values outside the set of collected data.

In linear regression models, the conditional mean of an independent variable given the dependent variable value is typically an affine function. In some cases, the median, or some other quantile of the conditional distribution of the independent variable given the dependent variable is a linear function of the dependent variable.

Non-linear regression is a type of regression analysis in which observed information is modeled by a non-linear function. The non-linear function is a combination of the model parameters and depends on an independent variable.

The environment information may comprise an alert information, for example alerting the user or a third party of the presence in the environment of the user of a specific chemical compound or that the concentration in CO is above a certain threshold. The alert information may be an audio and/or visual alert, for example via a display device.

The environment information may comprise a recommendation, for example, recommending to the user to go outside take some fresh air or on the contrary to reduce his sun exposition. The recommendation may also be to open the windows, for example if the concentration of specific chemical compound is greater greater than a threshold value. The recommendation may be an audio and/or visual recommendation.

For example, a user carrying out a physical activity outdoor, such as running, walking, biking, may be informed that the concentration of some chemical compound or pollen, in his environment is above a threshold value. Based on such information the user may be recommended to stop his physical activity. Furthermore, if the head mounted device comprises a geolocation module and a mapping module; the user may be informed of a better place around him, i.e. comprising a lower concentration of chemical compound or pollen nearby.

The head mounted device could furthermore provide guiding or navigation information based on the air composition, sounds and/or electromagnetic wave. For example, a user may wish to go for a walk in a less polluted environment as possible. The head mounted device using the geolocation and mapping data may provide navigation for the user based on such environmental criteria.

The environment information may comprise an activation parameter for example to activate a programmable lens or any other connected device such as home automation of a building such as light, window opening, ventilation activation.

The head mounted device according to the invention allows controlling exposure to the user to his environment vis-à-vis his comfort and his health such as retinal toxicity, chronobiological rhythms, migraine, respiratory hazards.

According to an embodiment of the invention, the head mounted device further comprises a geolocation module configured to determine geolocation data relative to the geolocation of the user of the head mounted device. The communication component is configured to send geolocation data together with the data indicative of the parameter of the environment to a mapping module.

Advantageously, having the device comprises a locating system, a timer and the specific sensors enable to measure the expected key parameters. It helps with simple hypothesis calculations, manager of the user's environment

The mapping module may be embedded in the head mounted device or may be in a distant entity.

According to an embodiment of the invention, the mapping module may comprise:
- a communication module configured to receive from the communication component data indicative of the parameter sense by the sensor together with geolocation data,
- a memory storing at least computer executable instructions and configured to store at least part of the received data; and
- a processor for executing the computer executable instructions stored in the memory. The computer executable instructions comprise instructions for processing the data indicative of the parameter of the environment of the user and the geolocation data to generate a mapping of the parameter of the environment.

Advantageously, the mapping module allows providing a mapping of the different data while the user is moving. For example, when the wearer is taking a walk the head mounted device may sense the air pollution and using the mapping module it may be possible to determine a precise mapping of air pollution not only around the user but also in along the route taken by the user.

According to an embodiment of the invention, the communication element of an environment analyzing module of the invention may be configured to receive from a distant entity, for example a mapping module, a mapping of a mapping parameter of the environment of the user, the mapping parameter relating to the air composition and/or the electromagnetic radiations and/or the sound of the environment of the user. The computer executable instructions further comprises instructions for processing the mapping upon generating environment information.

For example, the parameter sensed by the sensor and the mapping parameter relate to the air composition in the environment of the user.

The parameter sensed by the sensor and the mapping parameter may relate to the electromagnetic radiations in the environment of the user. For example radio and/or micro wave radiations or light radiation.

Furthermore, the parameter sensed by the sensor and the mapping parameter relate to the sound in the environment of the user.

Advantageously, such embodiment allows providing and/or updating a mapping of at least one environment parameter over a specific area.

The accuracy of such mapping may be further increased when established using a plurality of head mounted device according to the invention.

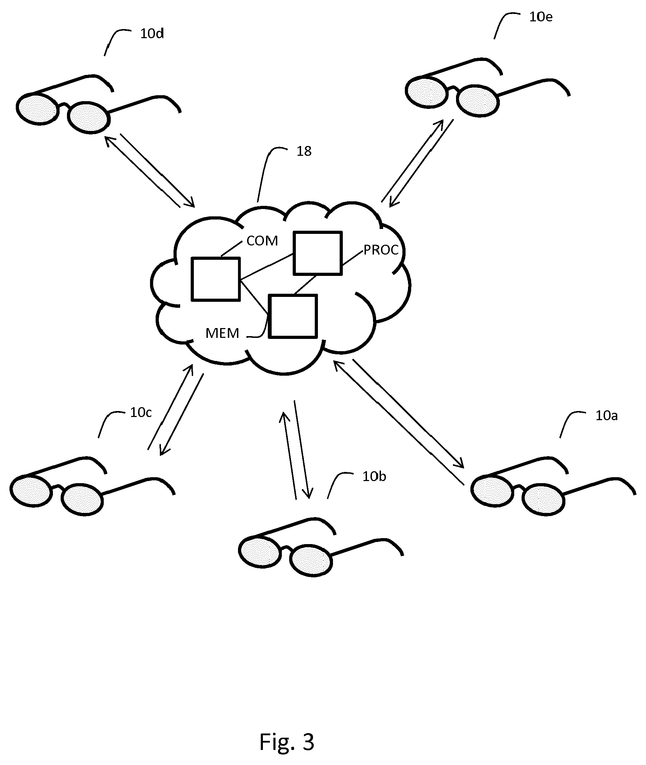

As illustrated on figure 3, the invention further relates to an environmental parameter mapping system comprising a plurality of head mounted devices 10 a to 10e, according to the invention.

Each head mounted device comprises a geolocation module configured to determine geolocation data relative to the geolocation of the user of the head mounted device and the communication component is configured to send geolocation data together with the data indicative of the parameter of the environment to a mapping module.

Furthermore each head mounted device is arranged to send geolocation data together with data indicative of the parameter of the environment to the mapping module.

The mapping module is configured to determine a mapping of the parameter over the zone or area in which the plurality of head mounted device is located.

The head mounted devices are arranged to communicate geolocation data and/or data indicative of the parameter of the environment to each other.

As represented on figure 3, all the head mounted devices may be configured to send the data to a single mapping module. Alternatively, the mapping system may comprise a plurality of mapping modules arranged to communicate with a central entity and/or between them.

Each head mounted device can communicate with one or more other head mounted device and/or environmental analyzing module and/or mapping module by way of the communications network, either directly or indirectly. The communication is preferably wireless, using Wifi or Bluetooth technologies. Even though illustrated as a single element in figure 3, the environmental mapping system can include other computing devices that provide services to the system and/or can represent multiple interconnected networks, which are not shown.

The network can be the Internet, the computing devices can be Web servers, file servers, media servers, etc. with which the head mounted device and/or environmental analyzing module and/or mapping module communicate via any of a number of known protocols, such as the hypertext transfer protocol (HTTP) or the hypertext transfer protocol secure (HTTPS).

The invention has been described above with the aid of embodiments without limitation of the general inventive concept.

Many further modifications and variations will suggest themselves to those skilled in the art upon making reference to the foregoing illustrative embodiments, which are given by way of example only and which are not intended to limit the scope of the invention, that being determined solely by the appended claims.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used. Any reference signs in the claims should not be construed as limiting the scope of the invention.

## Claims

1. A head mounted device comprising an environment sensing module configured to sense at least one parameter of the environment of the user of the head mounted device, the sensing module comprising at least:
- a sensor configured to sense a parameter of the environment of the user of the head mounted device,
- a communication component configured to send data sense by the sensor indicative of the parameter to an analyzing module,
wherein the parameter relates to the air in the direct environment of the user and/or the electromagnetic wave in the direct environment of the user and/or the acoustic wave in the direct environment of the user.

2. The head mounted device according to claim 1, wherein the sensor is adapted to sense the properties and/or composition of the air around the user, for example the presence and/or the quantity of allergen and/or of a specific chemical compound and/or of microparticles in the air around the user.

3. The head mounted device according to claim 2, further comprising a spectacle frame and wherein the sensor is mounted on the bridge and/or pad and/or brace bar of the spectacle frame.

4. The head mounted device according to any of the preceding claims, further comprising a geolocation module configured to determine geolocation data relative to the geolocation of the user of the head mounted device, and wherein the communication component is configured to send geolocation data together with the data indicative of the parameter of the environment to a mapping module.

5. The head mounted device according to claim 4, further comprising a mapping module comprising:
- a communication module configured to receive from the communication component data indicative of the parameter sense by the sensor together with geolocation data,
- a memory storing at least computer executable instructions; and
- a processor for executing the computer executable instructions stored in the memory, wherein the computer executable instructions comprises instructions for:
- processing the data indicative of the parameter of the environment of the user and the geolocation data to generate a mapping of the parameter of the environment.

6. The head mounted device according to any of the preceding claims, further comprising an environment analyzing module comprising:
- a communication element configured to receive from the communication component data indicative of the parameter sense by the sensor,
- a memory storing at least computer executable instructions; and
- a processor for executing the computer executable instructions stored in the memory, wherein the computer executable instructions comprises instructions for:
- processing the data indicative of the parameter of the environment of the user to generate environment information indicative of to the air composition and/or the electromagnetic radiations and/or the sound of the environment of the user.

7. The head mounted device according to claim 6, wherein the communication element is further configured to receive from a distant entity, for example a mapping module, a mapping of a mapping parameter of the environment of the user, the mapping parameter relating to the air composition and/or the electromagnetic radiations and/or the sound of the environment of the user and
the computer executable instructions further comprises instructions for:
- processing the mapping upon generating environment information.

8. The head mounted device according to claim 7, wherein the parameter sensed by the sensor and the mapping parameter relate to the air composition in the environment of the user.

9. The head mounted device according to claim 8, wherein the parameter sensed by the sensor and the mapping parameter relate to the electromagnetic radiations and/or to the sound in the environment of the user.

10. An environmental analyzing module configured to analyze at least one parameter of the environment of a user of a head mounted device according to any of the preceding claims, the module comprising:
- a communication element configured to receive from the communication component data indicative of the parameter sense by the sensor of the head mounted device,
- a memory storing at least computer executable instructions; and
- a processor for executing the computer executable instructions stored in the memory, wherein the computer executable instructions comprises instructions for:
- processing the data indicative of the parameter of the environment of the user to generate environment information indicative of to the air composition and/or the electromagnetic radiations and/or the sound of the environment of the user.

11. An environmental parameter mapping system comprising a plurality of head mounted devices according to any of the preceding claims, wherein each head mounted device further comprises a geolocation module configured to determine geolocation data relative to the geolocation of the user of the head mounted device, and wherein the communication component is configured to send geolocation data together with the data indicative of the parameter of the environment to a mapping module and each head mounted device is arranged to send geolocation data together with data indicative of the parameter of the environment to the mapping module, for example a common mapping module, configured to determine a mapping of the parameter over the zone in which the plurality of head mounted device is located.

12. Use of a mapping determined by an environmental parameter mapping system according to claim 10 or 11, for providing a service.

13. Method for monitoring at least one parameter of the environment of a user of a head mounted sensing device according to any of claims 1 to 9, the method comprising:
- an environment data receiving step S1, during which environment data indicative of at least one parameter of the environment of the user are received form the head mounted sensing device, the at least one parameter relates to the air in the direct environment of the user and/or the electromagnetic wave in the direct environment of the user and/or the acoustic wave in the direct environment of the user,
- an environment information generating step S2, during which an environment information is generated based at least on the received environment data.

14. The method according to claim 13, wherein the method further comprises prior to the environment data receiving step S1 a sensing step during which at least one parameter relating to the air in the direct environment of the user and/or the electromagnetic wave in the direct environment of the user and/or the acoustic wave in the direct environment of the user is sense by the sensor of the head mounted sensing device according to any of claims 1 to 9.

15. The method according to any of claim 13 or 14, wherein the method further comprises:
- a geolocation data receiving step during which geolocation data relative to the geolocation of the user are received, and
- a mapping generating step during which a mapping of the at least one parameter of the environment is generated based on the received environment data and geolocation data.
